(19) Europäisches Patentamt
European Patent Office
Office européen des brevets

(11) Veröffentlichungsnummer: **0 073 379**
**B1**

(12) # EUROPÄISCHE PATENTSCHRIFT

(45) Veröffentlichungstag der Patentschrift: **13.08.86**

(51) Int. Cl.⁴: **C 07 C 149/46**, C 07 C 148/00, C 08 K 5/36, H 01 B 1/12

(21) Anmeldenummer: **82107345.9**

(22) Anmeldetag: **13.08.82**

(54) **Neue Komplexsalze mit hoher elektrischer Leitfähigkeit.**

(30) Priorität: **26.08.81 DE 3133738**

(43) Veröffentlichungstag der Anmeldung: **09.03.83 Patentblatt 83/10**

(45) Bekanntmachung des Hinweises auf die Patenterteilung: **13.08.86 Patentblatt 86/33**

(84) Benannte Vertragsstaaten: **CH DE FR GB IT LI**

(56) Entgegenhaltungen:
**US-A-3 162 641**

**CHEMICAL ABSTARCTS, Band 95, Nr. 10, 7. September 1981, Seite 11, Nr. 81678p, Columbus, Ohio, USA J. PECHERZ et al.: "Synthesis and electrical conductivity of TCNQ complex salts with polycations containing sulfur atoms in the main chain"**

(73) Patentinhaber: **BAYER AG, Konzernverwaltung RP Patentabteilung, D-5090 Leverkusen 1 Bayerwerk (DE)**

(72) Erfinder: **Jonas, Friedrich, Dr., Krugenofen 15, D-5100 Aachen (DE)**
Erfinder: **Hocker, Jürgen, Dr., Eichenweg 6, D-5060 Bergisch Gladbach 2 (DE)**
Erfinder: **Broich, Bruno, Dr., Althaustrasse 16, CH-8957 Spreitenbach (CH)**

**EP 0 073 379 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1986

**Beschreibung**

Komplexsalze aus dem 7.7.8.8.-Tetracyano-p-chinodimethananion $(TNCQ)^{\ominus}$

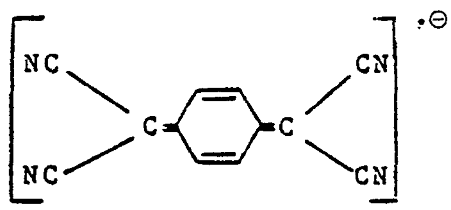

und anorganischen bzw. organischen Kationen (z.B. organischen Ammoniumionen) sind als elektrisch leitende Verbindungen bekannt [siehe US-Patent 3.162.641; J. Am. Chem. Soc. *98,* 3916 (1976)]. Diese Komplexe besitzen im allgemeinen die Zusammensetzung:

$$M^{\oplus} . TCNQ^{\ominus} . n\ TCNQ,$$

worin

$M^{\oplus}$ für ein anorganisches oder organisches Kation, z.B. für ein quaternäres Ammóniumion, Phosphoniumion oder Triarylsulfoniumion,

$TCNQ^{\ominus}$ für ein 7.7.8.8.-Tetracyanochinodimethanion und

n TCNQ für n neutrale TCNQ-Moleküle mit n=0 bis 4 stehen. Zur Erzielung hoher elektrischer Leitfähigkeiten ist n meist ≥ 1.

Diese Komplexe können durch Umsetzung von TCNQ mit organischen Kationiodiden hergestellt werden, z.B.:

$$4\ TCNQ + 3\ M^{\oplus}J^{\ominus} \rightarrow 2\ M^{\oplus} . TCNQ^{\ominus} . TCNQ + M^{\oplus}J_3^{\ominus}.$$

Hierbei wird ein TCNQ-Molekül durch lodid zum $TCNQ^{\ominus}$-Anion unter Freisetzung von lod reduziert, ohne daß lod in den Komplex eingebaut wird.

Überraschenderweise wurde nun gefunden, daß man Sulfoniumsalze mit TCNQ in geeigneten organischen Lösungsmitteln zu halogenhaltigen Komplexsalzen umsetzen kann.

Gegenstand der Erfindung sind neue, elektrisch leitende TCNQ-Komplexe der allgemeinen Formel I

$$S^{\oplus}R^1R^2R^3\ [X . TCNQ]^{\ominus} \qquad (I)$$

worin

$R^1$, $R^2$ und $R^3$ für gleiche oder verschiedene, lineare oder verzweigte Alkylgruppen mit bevorzugt 1 bis 12 C-Atomen, insbesondere bis 6 C-Atomen oder für gegebenenfalls substituierte Cycloalkylgruppen mit bevorzugt 5 bis 12 Ring-C-Atomen, insbesondere 5 bis 7 Ring-C-Atomen, oder für gegebenenfalls substituierte Aralkylgruppen mit bevorzugt 7 bis 12 C-Atomen, insbesondere 7 bis 9 C-Atomen stehen,

X für Halogen, vorzugsweise für lod und

TCNQ für 7.7.8.8.-Tetracyanochinodimethan stehen.

Die Verbindungen der allgemeinen Formel I können hergestellt werden, indem man Tetracyanochinodimethan und ein tert.-Sulfoniumsalz der Formel II

$$S^{\oplus}R^1R^2R^3\ X^{\ominus} \qquad (II)$$

worin

$R^1$, $R^2$, $R^3$ und X die in der Formel I angegebene Bedeutung haben, in einem geeigneten organischen Lösungsmittel bei Temperaturen zwischen 0°C und 120°C, vorzugsweise 20°C und 80°C zusammengibt. Bevorzugt werden äquimolare Mengen TCNQ und Sulfoniumsalz eingesetzt. Es kann aber auch ein Unterschuß bzw. Überschuß an Sulfoniumsalz verwendet werden.

Als geeignete organische Lösungsmittel können Acetonitril, Aceton oder Acetonitril-Methanol-Gemische, vorzugsweise Acetonitril verwendet werden. Die Reaktionsdauer kann zwischen 30 min. und mehreren Tagen, vorzugsweise zwischen 1 und 12 Std., liegen. Die Produkte fallen hierbei fest an und können durch einfaches Abfiltrieren aus den Lösungen isoliert werden.

Besonders geeignete Sulfoniumsalze sind Trimethylsulfoniumiodid, Triethylsulfoniumiodid, Trimethylsulfoniumbromid, Triethylsulfoniumbromid, Tripropylsulfoniumbromid und -iodid, Tributylsulfoniumbromid und -iodid, Dimethylethylsulfoniumbromid und -iodid, Cyclohexyldimethylsulfoniumiodid, Dicyclohexylmethylsulfoniumiodid, Benzyldimethylsulfoniumiodid, Tribenzylsulfoniumiodid, Tribenzylsulfoniumbromid.

Die erfindungsgemäßen TCNQ-Komplexe der Formel I zeigen hohe elektrische Leitfähigkeiten und können als elektrische Leiter, Halbleiter oder Photoleiter verwendet werden.

Weiterhin können die Verbindungen in Kunststoffe wie z.B. Polycarbonat eingearbeitet werden, um diese antistatisch auszurüsten.

## Beispiele

### Beispiel 1

Eine bei 60°C hergestellte Lösung von 5,3 g Trimethylsulfoniumiodid in 250 ml Acetonitril und 15 ml Ethanol wird zu einer siedenden Lösung von 4,2 g TCNQ in 400 ml Acetonitril gegeben. Man läßt langsam abkühlen und 12 Std. bei Raumtemperatur stehen. Die Lösung wird stark eingeengt, mit Methylenchlorid versetzt und die ausgefallenen Kristalle (II) abgesaugt. Es werden 8,3 g ≙ 98% d. Th. dunkelgrüne, glänzende Kristalle erhalten, die eine elektrische Leitfähigkeit von $2{,}22 \cdot 10^{-2}\Omega^{-1}cm^{-1}$ besitzen.

$$S(CH_3)_3^{\oplus}\,[J \cdot TCNQ]^{\ominus} \qquad \text{(II)}$$

### Beispiel 2

2,1 g TCNQ und 2,1 g Trimethylsulfoniumiodid werden in 300 ml Acetonitril zum Rückfluß (10 min.) erhitzt. Anschließend wird abgekühlt und eingeengt. Die ausgefallenen Kristalle werden abgesaugt und getrocknet. Es werden 2,9 g (69% d.Th.) dunkelgrüne Kristalle der Zusammensetzung $S(CH_3)^{\oplus}_3[J \cdot TCNQ]^{\ominus}$ erhalten.

| | | | | | |
|---|---|---|---|---|---|
| $C_{15}H_{13}JN_4S$ | Ber.: | 44,2% C | 3,2% H | 13,7% N | 31,1% J |
| | Gef.: | 44,8% C | 3,2% H | 14,1% N | 30,1% J. |

### Beispiel 3

2,1 g TCNQ und 2,2 g Dimethyl-ethyl-sulfoniumiodid werden in 300 ml Acetonitril zum Rückfluß (10 min.) erhitzt. Anschließend wird abgekühlt und das ausgefallene Produkt abgesaugt. Es werden 3,2 g (74% d.Th.) dunkelgrüne Kristalle der Zusammenzetzung $S(CH_3)_2C_2H_5^{\oplus}[J \cdot TCNQ]^{\ominus}$ erhalten.

### Beispiel 4

2,1 g TCNQ und 1,6 g Trimethylsulfoniumbromid werden, wie im Beispiel 3 beschrieben, umgesetzt. Es werden 1,8 g (49% d.Th.) dunkelgrüne Kristalle der Zusammensetzung $S(CH_3)_3^{\oplus}\,[Br \cdot TCNQ]^{-}$ erhalten.

**Patentansprüche**

1. Verbindungen der allgemeinen Formel I

$$S^{\oplus}R^1R^2R^3\,[X \cdot TCNQ]^{\ominus} \qquad \text{(I)}$$

worin

$R^1$, $R^2$ und $R^3$ für gleiche oder verschiedene Alkylgruppen, oder für gegebenenfalls substituierte Cycloalkylgruppen, oder für gegebenenfalls substituierte Aralkylgruppen mit vorzugsweise 7 bis 12 C-Atomen,

X für Halogen und

TCNQ für Tetracyanochinodimethan stehen.

2. Verfahren zur Herstellung der Verbindungen nach Anspruch 1, dadurch gekennzeichnet, daß man tert.-Sulfoniumhalogenide mit Tetracyanochinodimethan in einem organischen Lösungsmittel bei Temperaturen von 0°C bis 120°C umsetzt.

3. Verwendung der Verbindungen nach Anspruch 1 und 2 als elektrische Leiter, Halbleiter oder Photoleiter.

4. Verwendung der Verbindungen nach Anspruch 1 bis 3 zur antistatischen Ausrüstung von Kunststoffen.

**Revendications**

1. Composés de formule générale I

$$S^{\oplus}R^1R^2R^3\,[X \cdot TCNQ]^{\ominus} \qquad \text{(I)}$$

dans laquelle

$R^1$, $R^2$ et $R^3$ sont des groupes alkyle égaux ou différents, ou bien des groupes cycloalkyle éventuellement substitués, ou bien des groupes aralkyle éventuellement substitués ayant de préférence 7 à 12 atomes de carbone,

X est un halogène et

TCNQ désigne le tétracyanoquinodiméthane.

2. Procédé de production des composés suivant la revendication 1, caractérisé en ce qu'on fait réagir des halogénures de tertio-sulfonium avec du tétracyanoquinodiméthane dans un solvant organique à des températures de 0°C à 120°C.

3. Utilisation des composés suivant les revendications 1 et 2 comme conducteurs électriques, semiconducteurs ou photoconducteurs.

4. Utilisation des composés suivant les revendications 1 à 3, pour l'apprêtage antistatique de matières plastiques.

**Claims**

1. Compounds of the general formula I

$$S^{\oplus}R^1R^2R^3\ [X\ .\ TCNQ]^{\ominus} \qquad (I)$$

wherein

$R^1$, $R^2$ and $R^3$ represent identical or different alkyl groups, or optionally substituted cycloalkyl groups, or optionally substituted aralkyl groups with preferably 7 to 12 C atoms,

X represents halogen and

TCNQ represents tetracyanoquinodimethane.

2. Process for the preparation of the compounds according to Claim 1, characterised in that tert.-sulphonium halides are reacted with tetracyanoquinodimethane in an organic solvent at temperatures of 0°C to 120°C.

3. Use of the compounds according to Claim 1 and 2 as electrical conductors, semiconductors or photoconductors.

4. Use of the compounds according to Claim 1 to 3 for the antistatic finishing of plastics.